# EUROPÄISCHE PATENTSCHRIFT

(11) **EP 2 370 375 B1**
(45) Veröffentlichungstag und Bekanntmachung des Hinweises auf die Patenterteilung: **10.04.2019**
(21) Anmeldenummer: 09767958.3
(22) Anmeldetag: 30.11.2009
(51) Int. Cl.: C04B 40/00, C04B 20/00, G01H 13/00, G01N 33/38

(54) **VIBROAKUSTISCH WIRKSAMER ZUSCHLAGSTOFF**
VIBRO-ACOUSTICALLY EFFECTIVE AGGREGATE
MATÉRIAU GRANULAIRE À EFFET VIBRO-ACOUSTIQUE

(30) Priorität: 28.11.2008 DE 102008059471
(43) Veröffentlichungstag der Anmeldung: 05.10.2011
(73) Patentinhaber: Fraunhofer-Gesellschaft zur Förderung der angewandten Forschung e.V., 80686 München (DE)
(72) Erfinder: LEISTNER, Philip, 70569 Stuttgart (DE); DROTLEFF, Horst, 70374 Stuttgart (DE)
(74) Vertreter: Dörfler, Michael
(86) Internationale Anmeldenummer: PCT/EP2009/008532
(87) Internationale Veröffentlichungsnummer: WO 2010/060650

(56) Entgegenhaltungen:
- WO-A1-02/055280
- DE-A1-102004 005 912
- US-A1- 2008 179 993
- XAVIER PY, ET AL.: "Paraffin/porous-graphite-matrix composite as a high and constant powder thermal store material" INTERNATIONAL JOURNAL OF HEAT AND MASS TRANSFER, Bd. 44, Nr. 14, 1. Juli 2001 (2001-07-01), Seiten 2727-2737, XP002599262

## Beschreibung

### Technisches Gebiet

Die Erfindung betrifft einen vibroakustisch wirksamen granularen Zuschlagstoff für Materialien, wie z.B. für Beton, Gips und ähnliche Baustoffe, bestehend aus einer äußeren Hülle mit einer Füllung aus mindestens einem mittels innerlich oder äußerlich wirkender Felder, wie z.B. durch mechanische Spannungszustände, akustische, elektrische oder magnetische Felder, schaltbarem Phasenwechselmaterial und mindestens einer darin eingebetteten kompakten Masse, die in dieser Füllung je nach Aggregatzustand des Phasenwechselmaterials Schwingungen ausführt, wodurch der mechanische Zustand des umgebenden Materials charakterisiert und das vibroakustische Verhalten des umgebenden Materials beeinflusst wird.

### Stand der Technik

Beim Einsatz von Bauteilen aus Beton, Gips oder ähnlichen Baustoffen besteht der Bedarf, deren mechanische Festigkeit zu erfassen, zu analysieren und je nach Ergebnis die Festigkeit wieder zu erhöhen oder gar die Bauteile auszutauschen. Insbesondere festigkeitsrelevante Vorgänge im Inneren der Bauteile, wie z.B. entstandene und sich ausbreitende Mikrorisse, sind jedoch schwer zu detektieren. Da eine lokale Zerstörung der Bauteile mittels Kernbohrungen oder dergleichen möglichst vermieden werden soll, stehen nur wenige Verfahren zur zerstörungsfreien Bauteilprüfung und -analyse zur Verfügung. Diese unterscheiden sich nach Verfahren mit dauerhafter Detektion z.B. der von entstehenden oder wachsenden Mikrorissen ausgehenden akustischen Emissionen (engl. Acoustic Emission), die mit Körperschallsensoren erfasst werden. Bei entsprechend aufwändiger Anbringung vieler Sensoren und mittels geeigneter Auswerteverfahren lassen sich festigkeitsrelevante Vorgänge erkennen und auch lokalisieren. Neben dem sensorischen Aufwand und der begrenzten Präzision, ist es außerdem problematisch, dass. interne Reflexion und Streuung an Störkörpern wie Armierungen oder einfach an stofflichen Inhomogenitäten zu nicht auswertbaren Detektionsresultaten führen. Darüber hinaus beruhen diese Methoden unbedingt auf einer dauerhaften, zeitlich lückenlosen Detektion. Ansonsten werden einmalige Ereignisse nicht erfasst und die Information ist verloren. Auch die Verfahren, bei denen mittels Momentaufnahmen die genannten Vorgänge im Inneren erkannt werden können, weisen diese Nachteile auf. Sie verwenden z.B. eine gezielte Ultraschall- oder Radareinstrahlung in das Bauteil. Anhand des an der Oberfläche messbaren Reflexionsverhaltens wird auf bezüglich eines Referenzzustandes neu hinzugekommene Reflexionsstellen im Inneren geschlossen. Auch hier ist in bestimmten Fällen eine Lokalisierung möglich, allerdings ist oft die räumliche Auflösung zu gering, um die Auswirkungen kleiner Veränderungen, wie z.B. Mikrorisse, zu erfassen. Erschwerend kommt wiederum der apparative Aufwand hinzu. Unter Vernachlässigung der Frage des technischen Aufwandes sei noch auf eine zumindest konzeptionell entwickelte Technik zur Überwachung des inneren Festigkeitsverhaltens hingewiesen. Sie beruht auf Kombinationen von Sensoren, z.B. Drucksensoren, mit elektronischen Speicherbauteilen, bei denen das sensorisch erfasste Signal gespeichert und zu einem späteren Zeitpunkt ausgelesen wird. Eine an sich faszinierende Idee, allerdings ist die signaltechnische Erreichbarkeit eine ähnliche Herausforderung wie bei den oben genannten Verfahren. Weiterhin muss der Miniaturisierungsgrad beachtlich sein, damit die Sensor-Speicher-Elemente nicht selbst zu potentiellen Ausgangspunkten für bruchmechanische Ereignisse werden. Zweifellos existieren zu den vor genannten Verfahren und Methoden weitere Ausgestaltungen und Spielarten, ob mit anderen Sensoren oder als Kombinationen. Die prinzipiellen Einschränkungen bleiben davon weitgehend unberührt, wie es sich letztlich auch in der geringen praktischen Umsetzung widerspiegelt.

Aus der US 4,077,390 ist eine Wärmepackung bekannt, in dem eine unterkühlbare Natriumacetat-Lösung eingeschlossen ist. In der Natriumacetat-Lösung ist ein Aktivierungsstreifen angeordnet. Bei plötzlicher mechanischer Belastung erfolgt eine Kristallisation der Natriumacetat-Lösung. Dabei wird Wärme frei.

### Beschreibung - Austauschseiten

In der DE 196 51 384 A1 wird ein Verfahren vorgeschlagen, bei dem die Dichtheit einer Verpackung, bestehend aus Bodenteil und Deckel, bestimmt wird. Dazu ist in einem Hohlraum ein schwingfähiges Bauelement in einem Gas vorgegebener Viskosität eingeschlossen. Beim Eindringen eines Gases anderer Viskosität von außerhalb wird dann die Viskosität im Hohlraum beeinflusst. Somit werden auch die Schwingeigenschaften des schwingfähigen Elements beeinflusst. Im Ergebnis kann die Dichtheit der Verpackung jederzeit überprüft werden. Das schwingungsfähige Element kann generell zur Messung der Viskosität verwendet werden.

Aus der DE 101 02 250 A1 ist ein Latentwärmespeichermaterial auf Basis eines Phasenwechselmaterials bekannt. Diesem werden Federelemente zugegeben.

Aus der DE 101 46 895 A1 ist ein System zur Auswertung des Abklingverhaltens eines Prüfobjekts nach einer mechanischen Impulsanregung bekannt.

Aus der DE 10 2004 005 912 A1 ist ein schallabsorbierender Formkörper aus haufwerksporigem Beton bekannt. Dessen Poren haben einen mittleren Durchmesser von 0,2 bis 4 mm, wobei der Beton Mikrokapseln mit einem Polymer als Kapselwand und einem Kapselkern, bestehend überwiegend aus Latentwärmespeichermaterialien enthält. Aus dem Artikel "Paraffin/porous-graphite-matrix composite as a high and constant power thermal storage material", International Journal of Heat and Mass Transfer, Bd 44, Seiten 2727-2737, vom 1. Juli 2001 ist bekannt, eine Graphitmatrix als Aufnahme für Phasenwechselmaterial zu nutzen. Die Graphitmatrix dient zur verbesserten Wärmeleitung. Nach der Lehre dieses Artikels ist das Phasenwechselmaterial in die Graphitmatrix eingebracht. Dabei ist nicht anzunehmen, dass die Schwingfähigkeit wesentlich davon abhängt, ob das Phasenwechselmaterial im festen oder flüssigen Aggregatzustand vorliegt.

Die WO 02/055280 A1 zeigt ein Kunststoffteil, in das Phasenwechselmaterial eingearbeitet ist. Um bei der Verarbeitung des Kunststoffs das Phasenwechselmaterial nicht zu beschädigen, wird das Phasenwechselmaterial in schützende Trägermaterialteile eingebracht. Das Phasenwechselmaterial befindet sich dann in den Kapillarräumen dieser Trägermaterialteile.

In der US 2008/179993 A1 ist der Gedanke verwirklicht, durch Schwingungsanregungen die Stabilität eines Bauteils zu ermitteln. Dabei sind den zu untersuchenden Bauteilen granulare Zuschlagsstoffe in Form von piezoelektrischen Materialien beigemengt.

### Beschreibung

Der vorliegenden Erfindung liegt daher die Aufgabe zugrunde, die Nachteile des Standes der Technik zu überwinden und eine Möglichkeit anzugeben, die mechanische Festigkeit von Bauteilen einfach und ohne Schädigung des Bauteils einfach zu überwachen.

Diese Aufgabe wird durch die unabhängigen Ansprüche gelöst.

Unteransprüche geben vorteilhafte Weiterbildungen an.

Erfindungsgemäß wurde erkannt, dass hierzu ein granulärer Zuschlagsstoff für ein Bauteil aus einem Baustoff zu entwickeln ist. Der granulare Zuschlagsstoff ist dem Baustoff beizumischen. Der granulare Zuschlagsstoff ist aus nachfolgender Anordnung aufgebaut, das heißt, die einzelnen Granulate, werden von diesen Anordnungen gebildet. Damit ist es möglich, bei der Herstellung des Bauteils mehrere Anordnungen der nachfolgend näher beschriebenen Anordnungen in das Bauteil einzuarbeiten. Notwendig ist dabei lediglich, dass eine eventuell vorhandende Hülle der Anordnung Eigenschaften aufweist, welche zu einer guten Vermischung und guten Verbindung der Anordnungen mit dem Baustoff führt. Wie später noch genauer erläutert werden wird, kann durch die Einarbeitung der erfindungsgemäßen Anordnungen die Festigkeit des Bauteils gut untersucht werden.

In der Anordnung ist mindestens eine kompakte Masse in einem durch mechanische Einwirkung verfestigbarem Material oder Materialverbund derart anzuordnen, dass abhängig vom Verfestigungsgrad des verfestigbaren Materials die mindestens eine kompakte Masse Schwingungen ausführen kann. Bei dem verfestigbaren Material wird es sich in der Regel um ein unterkühltes Phasenwechselmaterial handeln, welches durch mechanische Einwirkung kristallisiert. Denkbar sind aber auch andere Stoffe, die durch mechanische Einwirkung verfestigbar sind. Dabei ist auch an einen Materialverbund verschiedener Stoffe zu denken, die durch mechanische Einwirkung miteinander reagieren und dadurch eine Verfestigung eintritt.

Unter einer Verfestigung ist jegliche Änderung zu verstehen, welche die Schwingungen der kompakten Masse beeinflussen können. Dies kann die Umwandlung einer Flüssigkeit in einen Festkörper bedeuten. Es ist aber auch möglich, dass eine Flüssigkeit lediglich an Zähigkeit beziehungsweise Viskosität zunimmt.

Dem granularen Zuschlagsstoff liegt die Überlegung zugrunde, dass eine kompakte Masse, die in einem verfestigbaren Material, beispielsweise in flüssigem Phasenwechselmaterial enthalten ist, durch äußere Einflüsse leicht zu Schwingungen angeregt werden kann. Wenn das verfestigbare Material hingegen etwa durch Kristallisationsprozesse in einen zähflüssigen oder festen Zustand übergegangen ist, so ist die Anregung von Schwingungen der kompakten Masse, wenn überhaupt, nur noch sehr schwer möglich. Versucht man nun durch äußere Einflüsse, beispielsweise durch Schall, die kompakte Masse zum Schwingen anzuregen, so hängt die sich tatsächlich einstellende Schwingung empfindlich vom Verfestigungsgrad des verfestigbaren Materials ab. Bei der kompakten Masse handelt es sich dabei in der Regel um einen festen Körper, der zu Schwingungen angeregt werden kann.

Die sich tatsächlich einstellenden Schwingungen der kompakten Masse sind als Schall messtechnisch gut zu detektieren. Somit kann relativ einfach auf den Verfestigungsgrad des verfestigbaren Materials, in der Regel des Phasenwechselmaterials, geschlossen werden. Der Verfestigungsgrad des verfestigbaren Materials ist bei geeigneter Auswahl des verfestigbaren Materials und gegebenenfalls eines geeigneten Temperaturbereichs ein Maß dafür, inwieweit in bestimmter Weise auf das verfestigbare Material eingewirkt worden ist. So tritt bei einer Reihe von Phasenwechselmaterialien die Kristallisation auch weit unterhalb der Phasenwechseltemperatur erst ein, wenn plötzlich starke mechanische Kräfte auf das Phasenwechselmaterial einwirken.

Der geschilderte granulare Zuschlagstoff kann somit dafür genutzt werden, festzustellen, ob auf das verfestigbare Material eine solche mechanische Einwirkung stattgefunden hat.

Die zusätzlich vorgesehene Hülle, welche das verfestigbare Material einfassen soll, hat lediglich die Funktion, die Anordnung zusammenzuhalten, da andernfalls das verfestigbare Material in flüssigem Zustand einfach wegfließen würde. Besteht diese Gefahr bei umgebenden Stoffen nicht, ist auch die Hülle nicht zwingend erforderlich.

Es ist auch möglich die kompakte Masse nicht in einem verfestigbaren Material anzuordnen, sondern in einer Matrix, die neben dem verfestigbaren Material oder Materialverbund zusätzlich weiteres Material enthält. Dies ist selbstverständlich nur dann machbar, wenn die Schwingungsfähigkeit der kompakten Masse weiterhin vom Verfestigungsgrad des verfestigbaren Materials abhängig ist.

Bevorzugt hat die Hülle eine Form nach Art einer Kugel. Damit kann die Anordnung kompakt gestaltet werden. Ferner ist es bei einer Kugel möglich, die Anordnung in jeder Hinsicht symmetrisch zu gestalten. Damit ist die Funktion der Anordnung unabhängig von ihrer Orientierung. Dies ist in der Praxis, insbesondere wenn die Anordnung in ein Bauteil eingearbeitet werden soll, wichtig, da die Einbaulage unbekannt ist und daher keinen Einfluss auf die Funktion haben darf.

Ein einfach realisierbarer und funktionstauglicher Aufbau der Anordnung kann mit einem mittleren Durchmesser der Anordnung von wenigen zehntel Millimetern bis zu wenigen Millimetern realisiert werden. Bevorzugt sind wenige Millimeter. Diese Größe eignet sich besonders gut für einen granularen Zuschlagsstoff für ein Bauteil.

Eine effiziente, insbesondere eine frequenzselektive Anregung von Schwingungen, wird erreicht, wenn das aus kompakter Masse und verfestigbarem Material bestehende Schwingsystem zu resonanten Schwingungen angeregt werden kann. Dabei ist bevorzugt eine Resonanzfrequenz zu wählen, die möglichst keiner Eigenschwingungsfrequenz (Eigenresonanz) des Bauteils entspricht und deren Wellenlänge kleiner als die Abmessungen des Bauteils ist Diese beiden Bedingungen verbessern unter anderem die spektrale Selektivität und die räumliche Lokalisierung.

Eine bevorzugte Ausführungsform des granularen Zuschlagsstoffs sieht vor, dass im Innern der Hülle Membrane oder Trennwände angeordnet sind. Damit ist es möglich, dass Erreichen oder Überschreiten einer vorgegebenen Grenzbelastung festzustellen, indem die Membranen oder Trennwände so ausgestaltet werden, dass sie beim Erreichen der Grenzbelastung reißen. Dies führt zwar zu einer gewissen Irreversibilität. Jedoch kann auf diese Weise relativ einfach sichergestellt werden, dass bei Erreichen der vorgegebenen Grenzbelastung eine Änderung des Verfestigungsgrads des verfestigbaren Materials eintritt. Auch wäre es denkbar, durch Membrane oder Trennwände mehrere unabhängig von einander schwingende kompakte schwingfähige, kompakte Massen vorzusehen, wobei die Einwirkungen auf das verfestigbare Material und damit der hervorgerufene Phasenwechsel abhängig von der Richtung der die Einwirkung auslösenden Kraft sein kann. Damit könnten richtungsselektiv wirkende granulare Zuschlagsstoffe bereit gestellt werden.

In einer weiteren bevorzugten Ausführungsform ist die Verfestigung, insbesondere die Phasenumwandlung des Phasenwechselmaterials, abhängig von der Frequenz einer auf das verfestigbare Material einwirkenden Schwingung. Durch eine solche Frequenzabhängigkeit kann erreicht werden, dass etwa die Frequenz, mit der bei Festigkeitsuntersuchungen die kompakte Masse zu Schwingungen angeregt wird, nicht zu einer unerwünschten Verfestigung im verfestigbaren Material führt. Ferner können verschiedene störende Einflüsse von einander getrennt werden, soweit diese Einflüsse mit unterschiedlicher Frequenz erfolgen, was jedoch im Allgemeinen der Fall sein dürfte. Besteht die kompakte Masse aus einem Material, das durch elektrische und/oder magnetische Felder zu Schwingungen angeregt werden kann, so steht für Festigkeitsuntersuchungen neben einer akustischen Anregung auch die elektrische und/oder magnetische Anregung zur Verfügung.

Es ist auch möglich in einer Anordnung verschiedene verfestigbare Materialien vorzusehen. Wenn gleich dies im Grunde stets möglich ist, so ist es insbesondere sinnvoll, dies bei einer Anordnung vorzusehen, die im Innern der Hülle Membrane oder Trennwände aufweist. Beispielsweise ist es möglich, zwei verschiedene verfestigbare Materialien in verschiedenen Bereichen der Anordnung vorzusehen, die beim Reißen einer Trennwand miteinander reagieren und dabei ihren Phasenzustand ändern. Somit ist ein sehr einfacher Nachweis des Reißens einer Trennwand möglich, welches von einem Überschreiten einer vorgegebenen Grenzbelastung hervorgerufen werden kann. Insbesondere bei dieser Ausgestaltungsform kann anstelle eines Phasenwechselmaterials auch ein anderes verfestigbares Material oder Materialverbund eingesetzt werden. Es genügt, dass auf unterschiedlichen Seiten der Trennwand oder der Membran unterschiedliche Stoffe vorhanden sind, die beim Reißen der Trennwand oder der Membran miteinander reagieren und sich dabei verfestigen.

Bei einer geeigneten Wahl des verfestigbaren Materials ist der der Verfestigungsgrad des verfestigbaren Materials durch äußere elektrische und/oder magnetische Felder beeinflussbar. Damit kann erreicht werden, dass die Schwingungsanregungen und damit die entsprechenden Festigkeitsuntersuchungen von einem äußeren Feld beeinflusst werden können. Dies erlaubt einen weiteren Bereich an Untersuchungen.

Wie bereits dargelegt können mit der Erfindung Bauteile aus einem Baustoff, in dem der granulare Zuschlagsstoff, beigemengt ist bereit gestellt werden. Dann kann der mechanische Zustand des Bauteils den Verfestigungsgrad des verfestigbaren Materials beeinflussen. Dies kann in einer Weise erfolgen, dass auch ein einmaliger mechanischer Zustand, also eine einmalige starke Belastung zu einer Änderung des Verfestigungsgrads des verfestigbaren Materials führt, welche beim Entfallen der Belastung nicht rückgängig gemacht wird. Damit sind im verfestigbaren Material Informationen über Belastungen, die das Bauteil geschädigt haben oder geschädigt haben könnten, gespeichert. Die Anordnungen dienen damit als vibroakustisch wirksame, granulare Zuschlagstoffe. Insbesondere können Bauteile bereit gestellt werden, bei denen eine plötzliche Änderung des mechanischen Spannungszustands des Bauteils den Verfestigungsgrad des verfestigbaren Materials ändert. Diese plötzlichen Änderungen des mechanischen Spannungszustands können insbesondere durch einen Mikroriss, einen Riss oder andere innere bruchmechanische Prozesse hervorgerufen werden. Auf diese Weise ist es also möglich, Bauteile bereit zu stellen, bei denen ein Mikroriss einfach festgestellt werden kann, indem die Schwingungsfähigkeit der kompakten Masse untersucht wird.

Zur Untersuchung eines geschilderten Bauteils wird bevorzugt das Bauteil zu Schwingungen angeregt und die Nachhallzeit und/oder Nachklingzeit und/oder Abklingzeit gemessen. Zur Unterscheidung sei auf die übliche Definition verwiesen, dass die Nachhallzeit für breitbandige Signale, z.B. Rauschen, gilt, während die Ab- oder Nachklingzeit für Einzeltöne oder zumindest schmalbandige Signale gilt. In allen Fällen ist die Zeit gemeint, die eine Schwingung nach Abschalten der Schwingungsquelle noch auftritt, bis sie eine bestimmte Amplitude unterschreitet.

Wie bereits angeführt, kann die Anregung mechanisch und/oder akustisch und/oder elektrisch und/oder magnetisch erfolgen.

Hierbei bieten sich insbesondere eine impulsartige oder eine harmonische Anregung an.

Die oben geschilderten Bauteile, welche die beschriebenen granularen Zuschlagsstoffe enthalten, weisen darüber hinaus den Vorteil auf, dass die durch das Bauteil erzielbare Schalldämmung und Schalldämpfung vom Verfestigungsgrad des verfestigbaren Materials abhängen. Dies gilt insbesondere bei Bauteilen aus leichten Baustoffen.

### Nähere Erläuterung

Ohne Einschränkung der Allgemeinheit wird die Erfindung anhand von Figuren nachfolgend näher beschrieben.

Die Figuren zeigen dabei:
Fig. 1
   Schematische Darstellung eines Bauteils 1 z.B. aus Beton, Gips oder ähnlichen Baustoffe mit im Inneren verteiltem vibroakustisch wirksamen granularen Zuschlagstoff 2, bestehend aus einer äußeren Hülle 3 sowie einer kompakten Masse 4, die in einem schaltbaren Phasenwechselmaterial 5 eingebettet ist.
Fig. 2
   Schematische Darstellung eines Bauteils 1 mit im Inneren verteiltem vibroakustisch wirksamen granularen Zuschlagstoff 2 und einem Mikroriss 6 als Ausgangspunkt für eine lokale, kurzzeitige Änderung des inneren mechanischen Spannungszustandes.
Fig. 3
   Schematische Darstellung der Veränderung, z.B. Versteifung, des schaltbaren Phasenwechselmaterial 5 infolge eines Mikrorisses 6 als Ausgangspunkt für eine lokale, kurzzeitige Änderung des inneren mechanischen Spannungszustandes.
Fig. 4
   Qualitative Änderung der Körperschallnachhallzeit eines Bauteils 1 infolge der Veränderung, z.B. Versteifung, des schaltbaren Phasenwechselmaterial 5 und der damit verbundenen verringerten Schwingfähigkeit der kompakten Masse 4.
Fig. 5
   Schematische Darstellung eines Bauteils 1 mit im Inneren verteiltem vibroakustisch wirksamen granularen Zuschlagstoff 2 und äußerlich anliegenden Sonden oder Elektroden 7 und entsprechenden Anschlüssen 8 zum Anlegen äußerer elektrischer oder magnetischer Felder zur Beeinflussung der Vibrationen des schwingfähigen Gebildes aus kompakter Masse 4 und schaltbarem Phasenwechselmaterial 5.
Fig. 6
   Vibroakustisch wirksamer granularer Zuschlagstoff 2 mit einer inneren Membran oder Trennwand 9.
Fig. 7
   Vibroakustisch wirksamer granularer Zuschlagstoff 2 mit mehreren inneren Membranen oder Trennwänden 9.
Fig. 8
   Vibroakustisch wirksamer granularer Zuschlagstoff 2 mit durch eine innere Membran oder Trennwand 9 getrennten verschiedenen Phasenwechselmaterialien 10 und 11 mit jeweils unterschiedlichen Eigenschaften.
Fig. 9
   Vibroakustisch wirksamer granularer Zuschlagstoff 2 mit einer flächenhaft ausgebildeten kompakten Masse 4, die je nach äußerem Feld seine Lage und damit ihre Schwingfähigkeit ändert.

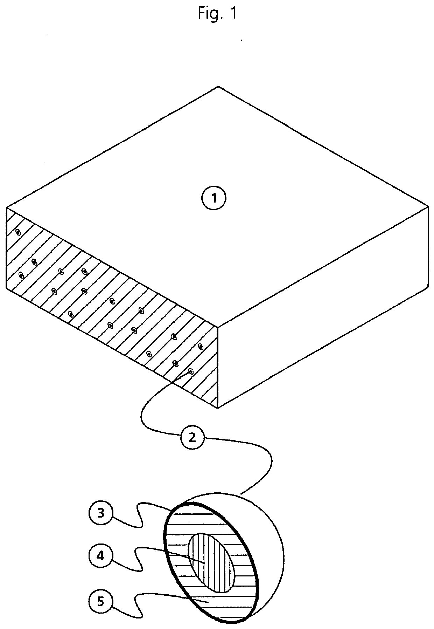

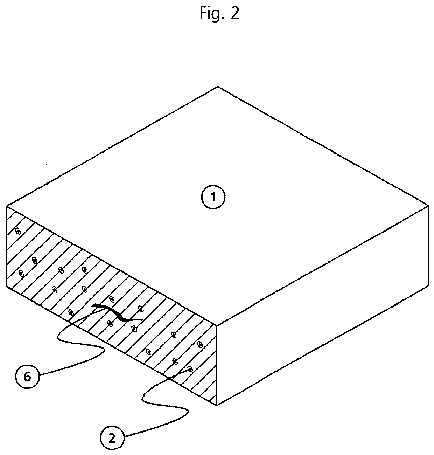

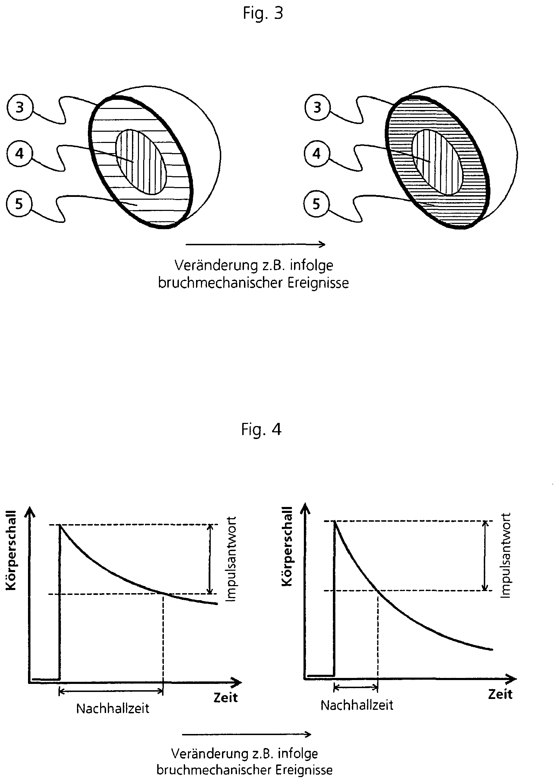

Die Verwendung des vibroakustisch wirksamen granularen Zuschlagstoffes 2 in einem Bauteil 1 erfolgt in gleicher Weise, wie dies für bekannte Zuschlagstoffe, wie z.B. Kies, Schotter in Beton, der Fall ist. Während der Bauteilherstellung wird er in geeigneter Menge untergemischt (Fig. 1) und somit zum festen Bestandteil des Bauteils 1. Dies bedeutet, dass die mechanische Stabilität des vibroakustisch wirksamen granularen Zuschlagstoffes 2 ausreichend bemessen sein muss und dass bei der Bauteilherstellung die erforderliche Vernetzung mit dem jeweiligen Bindemittel gewährleistet ist. Mechanische Stabilität und Benetzbarkeit sind daher immanente Eigenschaften der äußeren Hülle 3 des vibroakustisch wirksamen granularen Zuschlagstoffes 2. Die Hülle 3 ist mit mindestens einem schaltbaren Phasenwechselmaterial 5 gefüllt, dessen Aggregatzustand sich abhängig von innerlich oder äußerlich wirkenden Feldern ändert. Der Aggregatzustand kann also zwischen fest bzw. starr und flüssig bzw. elastisch und umgekehrt variieren. Diese Variation kann sowohl in Stufen oder aber stufenlos erfolgen. Unter innerlich wirkenden Feldern werden orts- und zeitabhängige mechanische Spannungszustände und deren Änderung im Bauteil 1 verstanden, wie sie z.B. im Zusammenhang mit Mikrorissen 6, Fig. 2, oder dergleichen auch kurzzeitig auftreten. Ähnliches gilt für akustische Felder und Emissionen. Im Phasenwechselmaterial 5 ist mindestens eine kompakte Masse 4 eingebettet, die in dieser Füllung je nach Aggregatzustand des Phasenwechselmaterials 5 Schwingungen ausführen kann, wodurch der mechanische Zustand des umgebenden Materials bzw. des Bauteils 1 charakterisiert wird. Fig. 3 veranschaulicht z.B. die Versteifung des Phasenwechselmaterials 5, ausgelöst durch die Änderung der mechanischen Spannung in der Nähe des Zuschlagstoffes 2 infolge eines Mikrorisses. Weitere Auslöser können die Überschreitung einer Belastungs- bzw. Verformungsgrenze der Hülle 3 sein. Fig. 4 zeigt qualitativ die Folgen der Versteifung anhand der Körperschallnachhallzeit. Im elastischen Zustand des schaltbaren Phasenwechselmaterials 5 kann die kompakte Masse 4 relativ frei schwingen, was zu einer Verlängerung der Körperschallnachhallzeit des Bauteils 1 führt. Nach der Versteifung des Phasenwechselmaterials 5 sind die Schwingungen stark eingeschränkt bis blockiert, so dass sich die Körperschallnachhallzeit reduziert. Das heißt, die geänderte Körperschallnachhallzeit resultiert aus der Schwingfähigkeit der kompakten Masse 4 im Phasenwechselmaterial 5. Die Schwingfähigkeit ist also als im Phasenwechselmaterial 5 gespeicherte Information über eine Änderung des mechanischen Spannungszustandes in der Nähe des Zuschlagstoffes 2 zu verstehen. Der vikroakustisch wirksame granulare Zuschlagstoff 2 vereint also Sensor- und Speicherfunktion in sich. Der verbleibende Schritt zur Auswertung des inneren Festigkeitszustandes ist demnach die Bestimmung der Körperschallnachhallzeit, z.B. mittels äußerer mechanischer bzw. akustischer Anregung und Messung der Nachhall-, Nachkling- oder Abklingzeit. Die Anregung kann impulsartig, harmonisch oder anderweitig erfolgen. Das Schwingsystem aus kompakter Masse 4 und Phasenwechselmaterial 5 ist nach Art eines Resonanzsystems aufeinander abzustimmen, so dass auch die Messung der Körperschallnachhallzeit auf die resultierende Resonanzfrequenz fokussiert werden kann. Dies erhöht die Selektivität und Genauigkeit des Verfahrens. Während für die kompakte Masse 4 zunächst keine besonderen stofflichen Vorgaben gelten, muss das Phasenwechselmaterial 5 die Fähigkeit besitzen, seinen Zustand in einem Bereich zwischen mindestens zwei Aggregatzuständen, z.B. elastisch und starr, verändern zu können. Dafür gibt es zahlreiche Beispiele, wie z.B. Paraffine oder Natriumacetatlösungen, bei denen durch anliegende mechanische Spannung ein Kristallisationsprozess ausgelöst wird, der zur Verfestigung führt. An dieser Stelle wird auch deutlich, dass eine äußere Impuls- oder Schwingungsanregung, z.B. im Zuge der Messung der Körperschallnachhallzeit, nicht ebenfalls zwangsläufig zu einer Verfestigung des Phasenwechselmaterials 5 führt, wie es bei plötzlicher Änderung des mechanischen Spannungszustandes infolge eines Mikrorisses der Fall ist. Die oben genannten Phasenwechselmaterialien können durchaus in Schwingungen versetzt werden, ohne dass der Kristallisationsprozess einsetzt. Es besteht also eine dynamische oder Frequenzselektivität des Phasenwechsels, die beim vibroakustisch wirksamen granularen Zuschlagstoff 5 ausgenutzt wird. Die beim Phasenwechsel meist auch frei werdende Wärme ist für hier vorliegende Verwendung ohne Bedeutung, während sie in der bisher üblichen Nutzung von Phasenwechselmaterialien den zentralen Zweck darstellt.

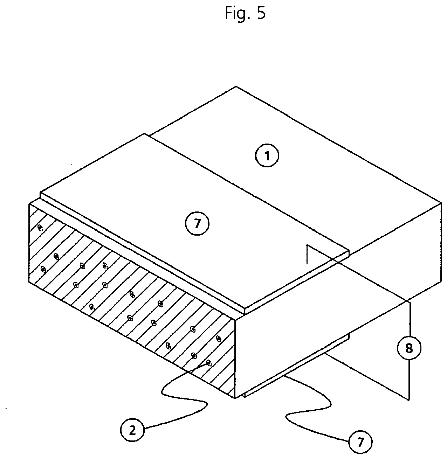

Die Schaltbarkeit des Phasenwechselmaterials 5 in Kombination mit der kompakten Masse 4 erstreckt sich, wie oben bereits erwähnt, auch auf äußere elektrische und magnetische Felder, die nach Art der Darstellung in Fig. 5 mit Sonden oder Elektroden 7 sowie mit entsprechenden Anschlüssen 8 angelegt werden. In diesem Zusammenhang gewinnt auch die Materialwahl der kompakten Masse 4 an Bedeutung, da sie auf die elektrischen oder magnetischen Feldkräfte reagieren können muss. Metallische oder ferromagnetische Stoffe sind jedoch in ausreichendem Maße bekannt. Bei außen am Bauteil 1 angelegtem Wechselfeld wird die kompakte Masse 4 im Phasenwechselmaterial 5 ebenfalls zu Schwingungen angeregt, so dass anstelle einer mechanischen oder akustischen Anregung zur Messung der Körperschallnachhallzeit auch diese Anregungsform genutzt werden kann. Dadurch wird jegliche Zerstörungsgefahr an der Oberfläche des Bauteils 1 ausgeschlossen.

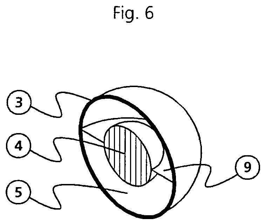

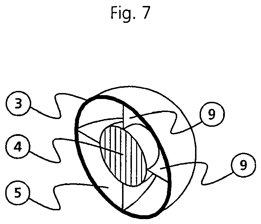

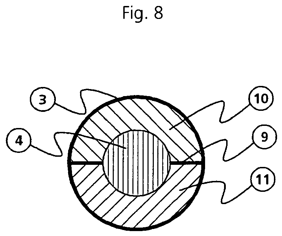

Zusätzlich zur bisher Funktionalität können weitere Ausgestaltungen des vibroakustisch wirksamen granularen Zuschlagstoffes 2 vorteilhaft verwendet werden. So wurde bereits die Überschreitung von Belastungs- oder Verformungsgrenzen der Hülle 3 als Auslöser für die Zustandsänderung des Phasenwechselmaterials 5 genannt. Sollte dies z.B. aus praktischen Gründen zu verhindern sein, können im Inneren der Hülle 3 Membranen oder Trennwände 9 vorgesehen werden, z.B. in der Art nach Fig. 6 und Fig. 7. Deren Verformungsreaktion wird so bemessen, dass sie bei einer definierten Soll-Verformung reißen oder dergleichen und somit die für das Phasenwechselmaterial 5 notwendige Änderung der Umgebungsbedingungen herbeiführen. Weiterhin können die inneren Membranen oder Trennwände 9 zwei verschiedene Phasenwechselmaterialien 10 und 11 mit unterschiedlichen Eigenschaften sowie mit mehreren oder auch einer gemeinsamen eingebetteten kompakten Masse 4 voneinander trennen, Fig. 8. Neben der unterschiedlichen Abstimmung des Resonanzsystems ergibt sich dadurch die Möglichkeit, dass bei Überschreitung einer definierten Soll-Verformung der Membran 9 und damit verbundener Zerstörung eine z.B. chemische Reaktion der Phasenwechselmaterialien 10 und 11 miteinander die resultierende Eigenschaft, z.B. Versteifung, des Gemisches bewirkt. Eine geeignet angeordnete Unterteilung mit mehreren Membranen 9 z.B. nach Art von Fig. 7 ließe sich sogar eine richtungsabhängige Reaktion herbeiführen und anschließend detektieren. Obgleich diese Reaktionen, wie Auf- oder Zerreißen der Membranen 9 zweifellos irreversible Prozesse darstellen, erfüllen sie den Zweck der Detektion und Speicherung von Informationen zum strukturellen Zustand des Bauteils 1. Daher sei auch auf die Gestaltungsvariante hingewiesen, dass auch die Hülle 3 selbst nach Überschreitung ihrer definierten Soll-Verformung perforiert und daraufhin das Phasenwechselmaterial 5 mit dem umgebenden Material des Bauteils 1 reagiert und seinen Zustand verändert.

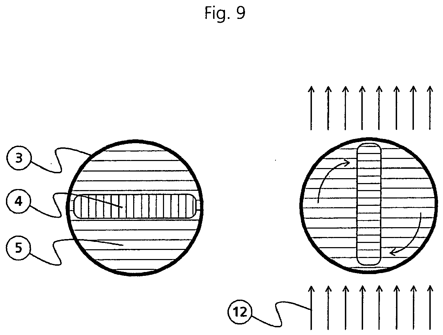

Unabhängig von den Ausgestaltungen ergibt sich aber noch ein weiterer wesentlicher Vorteil des vibroakustisch wirksamen granularen Zuschlagstoffes 2, der weniger in Richtung Charakterisierung des inneren Festigkeitszustandes zeigt, sondern vielmehr die Beeinflussbarkeit der akustischen Eigenschaften, wie z.B. Schalldämmung und sogar Schallabsorption, des Bauteils 1 mit sich bringt. Es ist bekannt, dass die Schalldämmung von Platten verändert werden kann, wenn sich im Plattenmaterial schwingfähige Gebilde, so genannte Oszillatoren, befinden (siehe z.B.: Locally Resonant Sonic Materials. Science 8/2000/ 289, pp. 1734 - 1736.). Dazu wurden z.B. Bleikügelchen mit einer Gummischicht ummantelt und in regelmäßiger Anordnung im Inneren einer Epoxydharz-Platte platziert. Die Wirkung dieser Oszillatoren führt zwar bei einzelnen Frequenzen zur Erhöhung der Schalldämmung, bei anderen Frequenzen jedoch zur Verringerung. Beim vibroakustisch wirksamen granularen Zuschlagstoff 2 lässt sich diese Erkenntnis vorteilhaft integrieren, mittels äußerer Felder aktivieren und insbesondere mit Hilfe der Veränderbarkeit des Phasenwechselmaterials 5 spektral optimieren. Fig. 9 zeigt beispielhaft, wie dies auch allein mit der durch ein äußeres Feld 12 bewirkten Lageänderung einer speziell geformten kompakten Masse 4 erfolgen kann. Die Vibrationen oder Oszillationen des schwingfähigen Gebildes aus kompakter Masse 4 und Phasenwechselmaterials 5 können also durch ein äußerlich anliegendes elektrisches oder magnetisches Wechselfeld im Frequenzbereich auf ein bestimmtes Band konzentriert und verstärkt werden. Zugleich können sie in anderen Frequenzbändern unterdrückt werden, um eine verringerte Schalldämmung zu vermeiden. Weiterhin kann mit einer dem Wechselfeld überlagerten konstanten Feldkraft die Resonanzfrequenz der Schwingungen nahezu stufenlos eingestellt werden.

All diese Vorteile beeinflussen nicht die Funktionalität des vibroakustisch wirksamen granularen Zuschlagstoffes 2, Informationen über bruchmechanische Ereignisse an beliebigen Stellen im Inneren von Bauteilen aus Beton, Gips oder ähnlichen Baustoffen zu erfassen, zu speichern und sie von außen zugänglich zu machen.

### Bezugszeichenliste

- 1: Bauteil
- 2: Vibroakustisch wirksamer Zuschlagstoff
- 3: Hülle
- 4: kompakte Masse
- 5: Phasenwechselmaterial
- 6: Mikroriss
- 7: Elektrode
- 8: Anschlüsse zum Anlegen elektrischer oder magnetischer Felder
- 9: Trennwand
- 10: Spezielles Phasenwechselmaterial
- 11: Spezielles Phasenwechselmaterial
- 12: Äußeres Feld

## Patentansprüche

1. Granularer Zuschlagstoff für die Beimengung zu einem Baustoff, **dadurch gekennzeichnet, dass** der Zuschlagsstoff aus Anordnungen (2) mit mindestens einer kompakten Masse (4) in einem durch mechanische Einwirkung verfestigbarem Material oder Materialverbund (5) in Form eines Phasenwechselmaterial (5), derart aufgebaut ist, dass das verfestigbare Material oder der Materialverbund (5) in einer Hülle (3) eingefasst sind und abhängig vom Verfestigungsgrad des verfestigbaren Materials oder Materialverbunds (5) die mindestens eine kompakte Masse (4) Schwingungen ausführen kann.

2. Granulärer Zuschlagstoff nach Anspruch 1, **dadurch gekennzeichnet, dass** die Anordnung (2) eine Form nach Art einer Kugel hat.

3. Granulärer Zuschlagstoff nach einem der vorhergehenden Ansprüche, **dadurch gekennzeichnet, dass** die Anordnung (2) einen mittleren Durchmesser von wenigen zehntel Millimetern bis wenigen Millimetern hat.

4. Granulärer Zuschlagstoff nach einem der vorhergehenden Ansprüche, **dadurch gekennzeichnet, dass** die Anordnung (2) so ausgebildet ist, dass die Anregung von Schwingungen mit einer Resonanzfrequenz möglich ist.

5. Granulärer Zuschlagstoff nach einem der vorhergehenden Ansprüche, **dadurch gekennzeichnet, dass** im Innern der Anordnung (2) oder der Hülle (3) Membranen oder Trennwände (9) angeordnet sind.

6. Granulärer Zuschlagstoff nach einem der vorhergehenden Ansprüche, **dadurch gekennzeichnet, dass** die Verfestigung abhängig von der Frequenz einer auf das verfestigbare Material oder den Materialverbund (5) einwirkenden Schwingung ist.

7. Granulärer Zuschlagstoff nach einem der vorhergehenden Ansprüche, **dadurch gekennzeichnet, dass** der feste Körper (4) aus einem Material besteht oder ein Material enthält, das durch elektrische und/oder magnetische Felder (12) zu Schwingungen angeregt werden kann.

8. Granularer Zuschlagstoff nach einem der vorhergehenden Ansprüche, **dadurch gekennzeichnet, dass** der Verfestigungsgrad des verfestigbaren Materials oder Materialverbunds (5) durch äußere elektrische und/oder magnetische Felder (12) beeinflussbar ist.

9. Bauteil (1) aus einem Baustoff, in den granulärer Zuschlagsstoff nach einem der vorhergehenden Ansprüche so beigemengt ist, dass der mechanische Zustand des Bauteils (1) den Verfestigungsgrad des verfestigbaren Materials oder Materialverbunds (5) beeinflusst.

10. Bauteil nach Anspruch 9, **dadurch gekennzeichnet, dass** eine plötzliche Änderung des mechanischen Spannungszustand des Bauteils (1), insbesondere hervorgerufen durch einen Mikroriss (6), einen Riss oder andere innere bruchmechanische Prozesse den Verfestigungsgrad des verfestigbaren Materials oder Materialverbunds (5) ändert.

11. Verfahren zur Untersuchung eines Bauteils (1) nach einem der Ansprüche 9 oder 10, **dadurch gekennzeichnet, dass** das Bauteil (1) zu Schwingungen angeregt wird und die Nachhallzeit und/oder die Nachklingzeit und/oder die Abklingzeit gemessen wird.

12. Verfahren nach Anspruch 11, **dadurch gekennzeichnet, dass** die Anregung mechanisch und/oder akustisch und/oder elektrisch und/oder magnetisch erfolgt.

13. Verfahren nach einem der Ansprüche 11 oder 12, **dadurch gekennzeichnet, dass** die Anregung impulsartig oder harmonisch erfolgt.

14. Verwendung eines Bauteils (1) nach einem der Ansprüche 9 oder 10 als Bauteil mit schaltbarer Schalldämmung und/oder Schalldämpfung.

## Claims

1. Granular aggregate for mixing with a building material, **characterized in that** the aggregate is made up of arrangements (2) comprising at least one compact mass (4) in a material or material composite (5) in the form of a phase-change material (5) that can be solidified by mechanical action, in such a way that the solidifiable material or the material composite (5) is enclosed in a shell (3) and, depending on the degree of solidification of the solidifiable material or material composite (5), the at least one compact mass (4) can perform vibrations.

2. Granular aggregate according to Claim 1, **characterized in that** the arrangement (2) has a shape like a sphere.

3. Granular aggregate according to one of the preceding claims, **characterized in that** the arrangement (2) has an average diameter of a few tenths of a millimetre to a few millimetres.

4. Granular aggregate according to one of the preceding claims, **characterized in that** the arrangement (2) is formed such that the excitation of vibrations at a resonant frequency is possible.

5. Granular aggregate according to one of the preceding claims, **characterized in that** membranes or dividing walls (9) are arranged in the interior of the arrangement (2) or the shell (3).

6. Granular aggregate according to one of the preceding claims, **characterized in that** the solidification is dependent on the frequency of a vibration acting on the solidifiable material or the material composite (5) .

7. Granular aggregate according to one of the preceding claims, **characterized in that** the solid body (4) consists of a material or contains a material that can be excited to perform vibrations by electric and/or magnetic fields (12).

8. Granular aggregate according to one of the preceding claims, **characterized in that** the degree of solidification of the solidifiable material or material composite (5) can be influenced by external electric and/or magnetic fields (12).

9. Component (1) of a building material into which granular aggregate according to one of the preceding claims has been mixed such that the mechanical state of the component (1) influences the degree of solidification of the solidifiable material or material composite (5).

10. Component according to Claim 9, **characterized in that** a sudden change in the mechanical state of stress of the component (1), in particular caused by a microcrack (6), a crack or other internal fracture-mechanical processes, changes the degree of solidification of the solidifiable material or material composite (5).

11. Method for investigating a component (1) according to either of Claims 9 and 10, **characterized in that** the component (1) is excited to perform vibrations and the reverberating time and/or the resonating time and/or the decay time is measured.

12. Method according to Claim 11, **characterized in that** the excitation takes place mechanically and/or acoustically and/or electrically and/or magnetically.

13. Method according to either of Claims 11 and 12, **characterized in that** the excitation takes place in a pulsed or harmonic manner.

14. Use of a component (1) according to either of Claims 9 and 10 as a component with switchable sound insulation and/or sound attenuation.

## Revendications

1. Agrégat granulaire à mélanger à un matériau de construction, **caractérisé en ce que** l'agrégat est élaboré à partir de structures (2) comprenant au moins une masse compacte (4) dans un matériau ou composite de matériaux (5), solidifiable par action mécanique, qui se présente sous la forme d'un matériau à changement de phase (5) de sorte que le matériau ou composite de matériaux (5) solidifiable est enfermé dans une enveloppe (3) et l'au moins une masse compacte (4) peut effectuer des oscillations en fonction du degré de solidification du matériau ou composite de matériaux (5) solidifiable.

2. Agrégat granulaire selon la revendication 1, **caractérisé en ce que** la structure (2) a la forme d'une bille.

3. Agrégat granulaire selon l'une des revendications précédentes, **caractérisé en ce que** la structure (2) a un diamètre moyen de quelques dixièmes de millimètres à quelques millimètres.

4. Agrégat granulaire selon l'une des revendications précédentes, **caractérisé en ce que** la structure (2) est conçue pour permettre l'excitation d'oscillations à une fréquence de résonance.

5. Agrégat granulaire selon l'une des revendications précédentes, **caractérisé en ce que** des membranes ou des parois de séparation (9) sont disposées à l'intérieur de la structure (2) ou de l'enveloppe (3).

6. Agrégat granulaire selon l'une des revendications précédentes, **caractérisé en ce que** la solidification dépend de la fréquence d'une oscillation agissant sur le matériau ou composite de matériaux (5) solidifiable.

7. Agrégat granulaire selon l'une des revendications précédentes, **caractérisé en ce que** le corps solide (4) est en un matériau ou contient un matériau pouvant être excité par des champs électriques et/ou magnétiques (12) de manière à osciller.

8. Agrégat granulaire selon l'une des revendications précédentes, **caractérisé en ce que** le degré de solidification du matériau ou du composite de matériaux (5) solidifiable peut être influencé par des champs électriques et/ou magnétiques (12) extérieurs.

9. Composant (1) en un matériau de construction dans laquel un agrégat granulaire selon l'une des revendications précédentes est ajouté de sorte que l'état mécanique du composant (1) influence le degré de solidification du matériau ou composite de matériaux (5) solidifiable.

10. Composant selon la revendication 9, **caractérisé en ce qu'**un changement soudain de l'état de contrainte mécanique du composant (1), notamment dû à une microfissure (6), à une fissure ou à d'autres processus mécaniques de fracture interne, modifie le degré de solidification du matériau ou composite de matériaux (5) solidifiable.

11. Procédé d'inspection d'un composant (1) selon l'une des revendications 9 ou 10, **caractérisé en ce que** le composant (1) est excité de manière à osciller et **en ce que** le temps de réverbération et/ou le temps de résonance et/ou le temps d'amortissement sont mesurés.

12. Procédé selon la revendication 11, **caractérisé en ce que** l'excitation est effectuée par des moyens mécaniques et/ou acoustiques et/ou électriques et/ou magnétiques.

13. Procédé selon l'une des revendications 11 ou 12, **caractérisé en ce que** l'excitation est effectuée de manière impulsionnelle ou harmonique.

14. Utilisation d'un composant (1) selon l'une des revendications 9 ou 10 comme composant à isolation phonique et/ou isolation acoustique commutable.
